# EUROPEAN PATENT SPECIFICATION

(11) **EP 1 725 871 B1**
(45) Date of publication and mention of the grant of the patent: **02.03.2011**
(21) Application number: 05718410.3
(22) Date of filing: 17.03.2005
(51) Int. Cl.: G01N 33/548

(54) **SEPARATION OF UNCONJUGATED AND CONJUGATED SACCHARIDES BY SOLID PHASE EXTRACTION**
TRENNUNG VON KONJUGIERTEN UND UNKONJUGIERTEN SACCHARIDEN MITTELS FESTPHASENEXTRAKTION
SEPARATION DE SACCHARIDES CONJUGUES ET NON CONJUGUES PAR EXTRACTION SUR PHASE SOLIDE

(30) Priority: 17.03.2004 GB 0406013
(43) Date of publication of application: 29.11.2006
(73) Proprietor: Novartis Vaccines and Diagnostics S.r.l., 53100 Siena (SI) (IT)
(72) Inventor: BARDOTTI, Angela, I-53100 Siena (IT); RICCI, Stefano, I-53100 Siena (IT); PROIETTI, Daniela, I-53100 Siena (IT)
(74) Representative: Marshall, Cameron John
(86) International application number: PCT/IB2005/000946
(87) International publication number: WO 2005/090985

(56) References cited:
- EP-A- 0 462 794
- BARDOTTI A A ET AL: "Quantitative determination of saccharide in Haemophilus influenzae type b glycoconjugate vaccines, alone and in combination with DPT, by use of high-performance anion-exchange chromatography with pulsed amperometric detection." VACCINE. 3 APR 2000, vol. 18, no. 19, 3 April 2000 (2000-04-03), pages 1982-1993, XP004202493 ISSN: 0264-410X
- LYNGBY JANNE ET AL: "Quantification of lipopolysaccharides in outer membrane vesicle vaccines against meningococcal disease. High-performance liquid chromatographic determination of the constituent 3-hydroxy-lauric acid" March 2002 (2002-03), BIOLOGICALS, VOL. 30, NR. 1, PAGE(S) 7-13 , XP009048600 ISSN: 1045-1056 abstract

## Description

### TECHNICAL FIELD

This invention concerns the analysts and quality control of vaccines that include saccharides (*e.g.* bacterial capsular saccharides), and especially those where the saccharides, arc conjugated to a carrier.

### BACKGROUND OF THE INVENTION

Immunogens comprising capsular saccharide antigens conjugated to carrier proteins are well known in the art. Conjugation converts T-independent antigens into T-dependent antigens, thereby enhancing memory responses and allowing protective immunity to develop, and the prototype conjure vaccine was for *Haemophilus influenzae* type b (Hib) [*e.g.* sec chapter 14 of ref. 1]. Since the Hib vaccine, conjugated saccharide vaccines for protecting against *Neisseria meningitidis* (meningococcus) and against *Streptococus pneumoniae* (pneumococcus) have been developed. Other organisms where conjugate vaccines arc of interest are *Streptcoccus agalactiae* (group R streptococcus) [2]*, Pseudomonas, aeruginosa* [3] and *Staphylococcus aureus* [4].

Conjugate vaccines for *N.meningitidis* serogroup C have been approved for human use, and include Menjugate™ [5], Meningitec™ and NeisVac-C™. Mixtures of conjugates from each of serogroups A, C, W135 and Y have been reported [*e.g.* refs. 6.9], including the Menaetra™ product. Other mixtures of conjugated antigens include: (i) meningococcal A/C mixtures [10,11]; (ii) the PrevNar™ product [12] containing seven pneumococcal conjugates; (iii) mixed meningococcal and Hib conjugates [13,14]; and (iv) combined meningococcal, pneumococcal and Hib conjugates [15].

Problems when dealing with conjugate vaccines include stability and batch-to-batch consistency. In Hib vaccines, for instance, catalytic depolymerisation of the saccharide has been reported [16], and conjugate of the serogroup A meningococcus capsule are readily hydrolysed [17]. Instability of conjugates undesirably leads to to reduction in effective dose of immunogenic conjugate over time, variation between batches, and increased levels of uncharacterised breakdown products. References 18 & 19 discuss issues concerning stability testing of Hib conjugate vaccines.

Consequently, hydrolysis of the glycoconjugates to free (*i.e.* unconjugated) saccharide needs to be monitored in the formulated vaccines alone or when in combination with other vaccines. This analysis typically requires Reparation of the unconjugated saccharide from the conjugate followed by quantitative saccharide analysis. The need for separation raises a number of problems since conjugate vaccines, especially combination vaccines, (typically contain a number of components which may interfere with each other during the separation. Known methods of separation include ultrafiltration, hydrophobic chromatography and selective precipitation, but these techniques are slow and suffer from poor reproductibility. They are also usually less than quantitative in their separation and Frequently depend on the nature of the saccharide.

It is an object of the invention to provide modifications and improvements in the quality control of conjugate vaccines for assessing their stability and integrity. In particular, it is an object to provide improved separation techniques prior to quantitative analysis.

### DISCLOSURE OF THE INVENTION

The inventors have discovered that solid phase extraction (SPE) provides faster and more reproducible separation of conjugated saccharides from unconjugated saccharides, thereby allowing quantitative separation of these saccharides. Furthermore, it has been discovered that, compared with know techniques, solid phase extraction does not depend on the nature of the saccharide, which is particularly advantageous for combination vaccines. In addition, any adjuvant present does not interfere with unconjugated saccharide analysis by SPE. Consequently, the separation of conjugated and unconjugated saccharide using SPE may be advantageously combined with a quantitative conjugate analysis to provide improved quality control for conjugate vaccines. The SPE separation is compatible with existing quantitative conjugate analysis techniques, such as high performance anion exchange chromatography with pulsed amperometric detection (HPAEC-PAD).

According to a first aspect of the invention, solid phase extraction is used to separate conjugated saccharide from unconjugated saccharide. Thus the invention provides a method of separating a conjugated saccharide component in a sample from an unconjugated saccharide component in the sample, comprising the step of passing the sample through a solid phase extraction device. The invention also provides the use of a solid phase extraction device for separating a conjugated saccharide component in a sample from an unconjugated saccharide component in the sample.

In a second aspect, the invention provides a method of preparing a sample for analysis of its unconjugated saccharide content, comprising the step of passing the sample through a solid phase extraction devices to separate conjugated sacccharide from unconjugated saccharide. The invention also provides a method of analysing a sample's unconjugated saccharide content, comprising the steps of (i) passing the sample through a solid phase extraction device to obtain a specimen comprising separated unconjugated saccharide and (ii) analysing the specimen's saccharide content to give the unconjugated saccharide content of the sample.

In a third aspect, the invention provides a method of releasing a vaccine for use hy physicians, comprising the steps of: (a) manufacturing a vaccine comprising a conjugated saccharide; (b) analyzing the vaccine's unconjugated saccharide content by a method of analysis of the invention; and, if the results from steps (h) indicate a saccharide content acceptable for clinical use, (c) releasing the vaccine for use by physicians. Step (b) may involve assessment of minimum saccharide concentration (*e.g.* between 1-20 µg of total saccharide), assessment of unconjugated:conjugated saccharide ratio (*e.g.* <20% of the total saccharide by weight is unconjugated saccharide, preferably ≤ 10%, ≤5%, *etc*). Step (b) may he performed on a package vaccine, or may be performed on a bulk vaccine prior to packaging. Where the vaccine is a combination vaccine, step (b) may be performed in respect of an individual type of saccharide, or of all types of saccharide.

### Methods of Analysis

Methods of analysing the unconjugated or conjugated saccharide content typically require separation of the unconjugated saccharide from the conjugated saccharide. Consequently, the analytical methods of the invention typically comprise the steps of (i) an initial preparation step of passing the sample through a solid phase extraction device to separate conjugated saccharide from unconjugated saccharide in the sample, followed by (ii) analysing the saccharide content of the separated unconjugated saccharide to obtain the unconjugated saccharide content of the sample. The present invention also provides a method of preparing a sample for analysis hy currying out step (i).

### Calculation and Measurement

In general, samples can be analysed in several ways using the invention. Unconjugated saccharide content can be measured *e.g.* to check for incomplete conjugation, or to follow conjugate hydrolysis by monitoring increasing free saccharide over time. In addition, by measuring total (*i.e.* unconjugated and conjugated) saccharide content in a sample, the ratio of free saccharide to total saccharide can be assessed (if necessary in respect of each saccharide), which can be used for regulatory or quality control purposes. In general, it is desirable to ensure that a vaccine includes <25% (*e.g.* <20%, <15%, <10% *etc*.) of each saccharide in free form. High levels of free saccharides mean a lower immunogenic dose of conjugate.

As an alternative, or in addition, to measuring the unconjugated saccharide content, the conjugated saccharide content can also measured *e.g.* to check for incomplete conjugation, or to follow conjugate hydrolysis by monitoring increasing free saccharide over time. Since the total saccharide content - unconjugated saccharide content + conjugated saccharide content, the unknown parameter may be determined from the two known parameters.

For example, by measuring the conjugated saccharide content and the total saccharide content it is possible to calculate the unconjugated saccharide content. Thus, measuring the conjugated saccharide content and total saccharide content constitutes a method of analysing the unconjugated saccharide content of a sample. It is, however, preferred to measure the unconjugated saccharide content directly rather than calculating it. However, if the separated conjugated saccharide is present in the effluent of the solid phase extraction device, it may be more convenient to calculate the unconjugated saccharide content rather than eluting the unconjugated saccharide retentate from the solid phase extraction device.

Thus unconjugated saccharide can be separated from conjugated saccharide and can be separately analysed, thereby allowing a determination of the amount of unconjugated material in a composition. However, comparing the unconjugated or conjugated amount to the total amount is easier than separately analysing the unconjugated and conjugated amount after separation, since one of the unconjugated and the conjugated saccharide will be retained on the solid phase extraction device.

Preferably, the method of analysis of the invention comprises the step of analysing the saccharide content of the separated unconjugated saccharide to give the unconjugated saccharide content of the sample.

The method of analysis preferably comprises the step of measuring the total saccharide content of the sample. Typically, this step will involve a preparative step of dividing the sample, one portion of the same being analysed for unconjugated saccharide content, another portion being analysed for total saccharide content.

Alternatively, the unconjugated saccharide content may be compared against a known total saccharide content of the sample or a standard saccharide content of the sample (*e.g.* the calculated amount of unconjugated saccharide present after a known period of time), instead of measuring the total saccharide content. In this embodiment, therefore, the method of analysis comprises the step of comparing the unconjugated saccharide content against a known total saccharide content of the sample or a standard saccharide content of the sample.

### Separating Different Types of Saccharide

The conjugated saccharides in the sample typically comprise saccharide antigens conjugated to carrier proteins. Frequently, the saccharides derived from a particular pathogen comprise oligo- or polysaccharides having a distribution of lengths. The saccharides comprising this distribution are all considered to be of the same "type". In a combination vaccine, *e.g.* a combination vaccine comprising a mixture of meningococcal capsular saccharides of serogroups C, W135 and Y, the vaccine will comprise a saccharide "type" associated with each component immunogen, *e.g.* a saccharide type associated with serogroup C immunogens, a saccharide type associated with serogroup W135 immunogens and a saccharide type associated with serogroup Y immunogens.

The methods of the invention separate unconjugated and conjugated saccharides and do not generally distinguish between different saccharide types, thus separating unconjugated and conjugated saccharide regardless of saccharide type.

However, the measurement of the unconjugated saccharide content of the individual types of saccharide in a combination vaccine is nevertheless possible. First, certain methods of quantitative glycoconjuate analysis discussed in more detail below allow the measurement of individual types of saccharide within combined glycoconjugate vaccines comprising more than one type of glycoconjugate immunogen, even where different saccharides share monosaccharide units. Consequently, the methods of the invention allow the analysis of the unconjugated saccharide content of a single or combined vaccine and, in respect of a combined vaccine comprising more than one type of glycoconjugate immunogen, for each individual type of saccharide or for all types of saccharide. The invention is particularly useful for analysing the unconjugated content of *N.meningitidis* serogroup C saccharide in a combination vaccine comprising more than one type of glycoconjugate immunogen, *e.g.* combination vaccines comprising:
- mixtures of conjugates from each of meningococcal serogroups C and Y;
- mixtures of conjugates from each of meningococcal serogroups C, W135 and Y;
- mixtures of conjugates from each of meningococcal serogroups A, C, W135 and Y; or
- mixtures of conjugates from meningococcal serogroups A and C.

Second, the sample or the separated unconjugated saccharide may undergo another separation step to separate different types of saccharide.

### Measuring the Saccharide Content of a Composition

As mentioned above, in addition to measuring the saccharide content of the separated unconjugated saccharide, the method of analysis of the present invention may optionally include the step of measuring the total (*i*.*e*. unconjugated and conjugated) saccharide content of an individual type of saccharide or the content of all types of saccharide. The total saccharide content may be measured by measuring the saccharide content of the sample before separation. Thus, the present invention may require the measurement of the saccharide content of the separated unconjugated saccharide, the separated conjugated saccharide or the sample before, after or at the same time as separation.

Methods for measuring the saccharide content of compositions are well known in the art. Typically, the saccharide is treated in order to depolymerise the saccharides to give their constituent monosaccharides prior to analysing the monosaccharide content. Typically, however, the methods of analysis of the invention will comprise the step of treating the composition to be analysed (*i*.*e*. separated unconjugated saccharide, separated conjugated saccharide or the sample before separation) in order to depolymerise the saccharides to give their constituent monosaccharides. Analysis of saccharide content can then proceed on the depolymerised mixture of released monosaccharides. Since the monosaccharide content is directly related to the content of saccharide in the composition to be analysed prior to depolymerisation, analysis of the saccharide content of depolymerised monosaccharides allows determination of the saccharide content of the composition.

Conditions for depolymerisation of saccharides to their constituent monosaccharides are known in the art and typically comprise acid or base hydrolysis. For example, the serogroup C saccharide can be hydrolysed for total saccharide content analysis by treatment with 100 mM HCl at 80°C for 2 hours [20]. Acid hydrolysis using HCl may also be used for the Hib saccharide and typical treatment involves addition of TFA to a final concentration of 0.3M and heating at 100°C for 2 hours. Acid hydrolysis using trifluoroacetic acid (TFA) can be used for hydrolysis of all of serogroups C, W135 and Y, with a slightly lower incubation temperature being preferred for serogroup C to avoid degradation of its sialic acid (90°C rather than 100°C). A typical TFA treatment involves addition of TFA to a final concentration of 2M, followed by heating to 90-100°C for 90 minutes. Acid hydrolysis using TFA can also be used for hydrolysis of MenA polysaccharide and typical treatment involves addition of TFA to a final concentration of 2M and heating at 100°C for 2 hours. Acid hydrolysis using TFA can also be used for hydrolysis of Hib saccharide and typical treatment involves addition of TFA to a final concentration of 4M and heating at 100°C for 2 hours [21]. Base hydrolysis using NaOH can also be used for hydrolysis of Hib saccharide [21].

After depolymerisation, saccharide hydrolysates may be dried *e.g.* using a vacuum drier.

After depolymerisation of a combination vaccine comprising more than one type of glycoconjugate immunogen, or the separated unconjugated saccharide from such a combined vaccine, the composition may contain mixed monosaccharides of different types. The quantities of these monosaccharides in the mixture are directly related to the quantities of saccharides in the original pre-hydrolysis composition, and so quantities of the starting saccharides can be determined by utilising the methods of the invention in the process for analysing the saccharide content of a composition disclosed in reference 22.

Progress of depolymerisation (*e.g.* to check for total hydrolysis to monosaccharides rather than partial hydrolysis to oligosaccharides) can be checked by measuring the degree of polymerisation (DP) in a mixture, using known t echniques *e.g.* NMR, mass spectrometry, *etc.*

Once the saccharide has been depolymerised to monosaccharides, the monosaccharides are quantified. Consequently, the methods of analysis of the invention therefore typically comprise the step of quantifying the monosaccharides obtained from the depolymerisation step.

Methods for quantifying monosaccharides, including glucose (*e.g.* for *N.meningitidis* serogroup Y saccharide), galactose (*e.g.* for *N. meningitidis* serogroup W135 saccharide), sialic acid (*e.g.* for *N.meningitidis* serogroup C saccharide), ribitol (*e.g.* for Hib saccharide) and mannosamine-6-P *(e.g.* for *N.meningitidis* serogroup A saccharide) are well known in the art.

Methods of quantification may be direct or indirect (*e.g.* they may involve derivatisation of the monosaccharides followed by an analysis that correlates with original monosaccharide content). If necessary, methods may involve separation of the different monosaccharides from each other, followed by separate analysis, and in such a case the actual measurement of monosaccharide content could be the same in each case, with specificity arising from the separation. It is preferred, however, to use methods which can analyse the saccharides in each other's presence, such that they do not need to be separated from each other before analysis. In addition, methods may be used for conjugated saccharides in which, after deconjugation, the carrier and the saccharide need not be separated. One preferred method is anion chromatography, and in particular high performance anion exchange chromatography (HPAEC), in particular with pulsed amperometric detection (PAD) [23,24]. HPAEC-PAD systems are provided by Dionex™ Corporation (Sunnyvale, CA) *e.g.* the BioLC™ system, using a column such as PA1 [10 µm diameter polystyrene substrate 2% crosslinked with divinylbenzene, agglomerated with 500 nm MicroBead quaternary ammonium functionalized latex (5% crosslinked)] or PA10 [10 µm diameter ethylvinylbenzene substrate 55% crosslinked with divinylbenzene, agglomerated with 460 nm MicroBead difunctional quaternary ammonium ion (5% crosslinked)]. These systems can quantitatively analyse individual saccharides within mixtures without the need for derivatisation or pre-analysis separation. For saccharide analysis, it may be desired to filter other compounds before entry to the column, and Dionex™ produces pre-column traps and guards for this purpose *e*.*g*. an amino trap for removing amino acids, a borate trap, *etc.*

An alternative method for quantifying monosaccharides within a depolymerised mixture is nuclear magnetic resonance (NMR). For ease of use and for high sensitivity, however, the chromatographic methods of the invention are preferred.

Once the monosaccharide content has been determined, it is straightforward to calculate the quantity of saccharides in the original composition.

The process of the invention is typically destructive. Rather than perform the process on a complete composition, therefore, it is more typical to take a sample from a composition of interest and then perform the analysis on the sample.

### Analysis of non-saccharide components

As well as analysing the content of saccharides in a composition, the method of analysis may include analysis of other components or properties *e.g.* osmolality, pH, degree of polymerisation for individual saccharides or conjugates, protein content (particularly for carrier proteins), aluminium content, detergent content, preservative content, *etc.*

The invention provides a method for preparing a vaccine composition, comprising a step of analysis of a sample according to the invention, including a step of pH measurement, followed by a step of adjusting the pH of the composition to a desired value *e.g.* between 6 and 8, or about 7.

The invention provides a method for packaging a vaccine, comprising the steps of: (a) manufacturing a bulk vaccine containing a conjugated saccharide; (b) analysing the unconjugated saccharide content in the bulk vaccine by a method of analysis of the invention; (c) optionally, analysing the bulk vaccine for pH and/or other properties; and, if the results from step (b) and (c) indicate that the bulk vaccine is acceptable for clinical use, (d) preparing and packaging the vaccine for human use from the bulk. Step (c) may involve (see above) assessment of minimum saccharide concentration, assessment of unconjugated:conjugated saccharide ratio, *etc.* Step (d) may involve packaging into unit dose form or in multiple dose form *e.g.* into vials or into syringes. A typical human dose for injection has a volume of 0.5ml.

Step (c) and/or (d) may be preceded by mixing the bulk vaccine with one or more further antigens *e.g.* with
- a capsular saccharide antigen from serogroup A of *N.meningitidis.*
- a capsular saccharide antigen from serogroup C *of N.meningitidis.*
- a capsular saccharide antigen from serogroup W135 of *N.meningitidis.*
- a capsular saccharide antigen from serogroup Y of *N.meningitidis.*
- a protein antigen from serogroup B of *N.meningitidis*
- preparations of *N.meningitidis* serogroup B microvesicles [25], 'native OMVs' [26], blebs or outer membrane vesicles [*e.g.* refs. 27 to 32 *etc.*].
- a saccharide antigen from *Haemophilus influenzae* type b
- an antigen from *Streptococcus pneumoniae,* such as polyvalent conjugated saccharide antigens [*e.g.* refs. 33 to 35].
- an antigen from hepatitis A virus, such as inactivated virus [*e.g.* 36, 37].
- an antigen from hepatitis B virus, such as the surface and/or core antigens [*e.g.* 37, 38].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 39 & 40]. Cellular pertussis antigens may be used.
- a diphtheria antigen, such as a diphtheria toxoid [*e.g.* chapter 13 of ref. 1] *e.g.* the CRM₁₉₇ mutant [*e.g.* 41].
- a tetanus antigen, such as a tetanus toxoid [*e.g.* chapter 27 of ref. 1].
- polio antigen(s) [*e.g.* 42, 43], such as IPV.

Such antigens may be adsorbed to an aluminium salt adjuvant (*e.g.* a hydroxide or a phosphate). Any further saccharide antigens are preferably included as conjugates.

### Further Steps

It may desirable, particularly prior to saccharide content analysis, to remove at least some non-saccharide compounds from the sample. Dionex^{™} produce pre-column traps and guards for this purpose, *e.g.* an amino trap for removing amino acids, a borate trap, *etc.*

It may also be desirable to remove at least some non-saccharide compounds from the sample prior to the step of separating conjugated saccharide from unconjugated saccharide. For example, when the vaccine is formulated with aluminium-containing adjuvants, solid phase extraction may typically be applied to the supernatant after centrifugation of the suspension.

After analysis of the saccharide content, the invention may include the further step of determining a characteristic of a saccharide, *e.g.* its DP (typically an average DP), its molecular weight, its purity, *etc.*

After amperometric and spectroscopic detectors, the separated unconjugated or conjugated saccharide may be coupled into a mass spectrometer, *e.g.* FAB/MS or ESI/MS.

### Analysis of Conjugated Saccharide Content

By measuring the conjugated saccharide content and the total saccharide content it is possible to calculate the unconjugated saccharide content. Similarly, by measuring the unconjugated saccharide content and the total saccharide content it is possible to calculate the conjugated saccharide content. Therefore, in addition to the above mentioned aspects of the invention, the invention also provides methods, and the products obtainable therefrom, directed to analysing a sample's conjugated saccharide content, *i.e.* the above methods and products (particularly of the second and third aspects of the invention, the method of packaging a vaccine, and the disclosures in respect of each of them thereafter) may be read wherein "conjugated saccharide" is substituted for "unconjugated saccharide" and "unconjugated saccharide" is substituted for "conjugated saccharide" (*e.g.* a method of analysing a sample's conjugated saccharide content, comprising the steps of (i) passing the sample through a solid phase extraction device to obtain a specimen comprising separated conjugated saccharide and (ii) analysing the specimen's saccharide content to obtain the conjugated saccharide content of the sample).

The invention therefore allows either direct or indirect analysis of the unconjugated saccharide content of a sample.

### Samples

The invention is particularly useful for analysing the unconjugated or conjugated saccharide content of a sample (*e.g.* a vaccine) or for preparing a sample for analysis of the unconjugated saccharide content of a sample (*e.g.* a vaccine). It is not essential to this embodiment that the invention contains any unconjugated or conjugated saccharide as the invention may be usefully employed to determine the presence or absence of unconjugated or conjugated saccharide. Moreover, a step of analysing the saccharide content of a sample or specimen which leads to a negative result, *i.e.* the absence of saccharide, is still a step of analysing the saccharide content of a sample or specimen.

However, it is preferred that the sample is suspected to contain (and preferably contains) conjugated saccharide or unconjugated saccharide. It is more preferred that the sample contains conjugated saccharide and is suspected to contain (and preferably contains) unconjugated saccharide.

The present invention is, however, more generally useful for separating conjugated saccharide from unconjugated saccharide in a sample (*e.g.* a vaccine). In this embodiment the sample preferably contains both conjugated saccharide and unconjugated saccharide.

The sample will generally be in aqueous solution.

As well as containing saccharides, samples to be analysed can include other materials. These may or may not be retained by the solid phase extraction device. Typically such components will not be retained.

The sample analyte may be a product to be tested prior to release (*e.g.* during manufacture or quality control testing), or may be a product to be tested after release (*e.g.* to assess stability, shelf-life, *etc.*).

Preferred samples are glycoconjugate vaccines, which may be single or combined (*e.g.* a combined glycoconjugated vaccine comprising more than one type of glycoconjugate immunogen).

### Conjugates

The conjugated saccharides are covalently linked saccharide-carrier conjugates. Covalent conjugation is used to enhance immunogenicity of saccharides by converting them from T-independent antigens to T-dependent antigens, thus allowing priming for immunological memory. Conjugation is particularly useful for paediatric vaccines and is a well known technique [*e.g.* reviewed in refs. 44 to 53]. Saccharides may be linked to carriers (*e.g.* proteins) directly [54, 55], but a linker or spacer is generally used *e.g.* adipic acid, β-propionamido [56], nitrophenyl-ethylamine [57], haloacyl halides [58], glycosidic linkages [59], 6-aminocaproic acid [60], ADH [61], C₄ to C₁₂ moieties [62], *etc.*

### Carrier Proteins in Conjugates

Typical carrier proteins in conjugates are bacterial toxins or toxoids, such as diphtheria toxoid or tetanus toxoid. The CRM₁₉₇ diphtheria toxin derivative [63-65] is the carrier protein in Menjugate™ and Meningitec™, whereas tetanus toxoid is used in NeisVac™. Diphtheria toxoid is used as the carrier in Menactra™. Other known carrier proteins include the *N.meningitidis* outer membrane protein [66], synthetic peptides [67,68], heat shock proteins [69,70], pertussis proteins [71,72], cytokines [73], lymphokines [73], hormones [73], growth factors [73], artificial proteins comprising multiple human CD4⁺ T cell epitopes from various pathogen-derived antigens [74] (*e.g.* N19 [75]), protein D from *H.influenzae* [76,77], pneumococcal surface protein PspA [78], iron-uptake proteins [79], toxin A or B from *C.difficile* [80], *etc.* Compositions may use more than one carrier protein *e.g.* to reduce the risk of carrier suppression, and a single carrier protein might carry more than one saccharide antigen [81]. Conjugates generally have a saccharide:protein ratio (w/w) of between 1:5 (*i.e.* excess protein) and 5:1 *(i.e.* excess saccharide).

### Saccharides in Conjugates

The conjugate saccharides may be polysaccharides (*e.g.* with a degree of polymerisation of >10, *e.g.* 20, 30, 40, 50, 60 or more) or oligosaccharides (*e.g.* with a degree of polymerisation of from 4 to 10). Oligosaccharides may be the result of depolymerisation and/or hydrolysis of a parent polysaccharide *e.g.* the analyte may be a saccharide-containing fragment of a larger saccharide. Preferred conjugate saccharides are capsular saccharides.

Even more preferred conjugate saccharides are bacterial capsular saccharides *e.g.* from *Neisseria meningitidis* (serogroups A, B, C, W135 or Y), *Streptococcus pneumoniae* (serotypes 4, 6B, 9V, 14, 18C, 19F, or 23F), *Streptococcus agalactiae* (types Ia, Ib, II, III, IV, V, VI, VII, or VIII), *Haemophilus influenzae* (typeable strains: a, b, c, d, e or f), *Pseudomonas aeruginosa, Staphylococcus aureus, etc.*

The *N.meningitidis* serogroup A capsule is a homopolymer of (α1→6)-linked *N-*acetyl-D-mannosamine-1-phosphate.

The *N.meningitidis* serogroup B capsule is a homopolymer of (α 2→8) linked sialic acids.

The *N.meningitidis* serogroup C capsular saccharide is a homopolymer of (α 2→9)-linked sialic acid (*N*-acetyl neuraminic acid, or 'NeuNAc'). Most serogroup C strains have O-acetyl groups at C-7 and/or C-8 of the sialic acid residues, but about 15% of clinical isolates lack these O-acetyl groups [82,83]. The acetylation does not seem to affect protective efficacy (*e.g.* unlike the Menjugate™ product, the NeisVac-C™ product uses a de-O-acetylated saccharide, but both vaccines are effective).

The *N.meningitidis* serogroup W135 saccharide is a polymer of sialic acid-galactose disaccharide units [→4)-D-Neu*p*5Ac(7/9OAc)-α-(2→6)-D-Gal-α-(1→]. Like the serogroup C saccharide, it has variable O-acetylation, but at sialic acid 7 and 9 positions [84].

The *N.meningitidis* serogroup Y saccharide is similar to the serogroup W135 saccharide, except that the disaccharide repeating unit includes glucose instead of galactose galactose [→4)-D-Neu*p*5Ac(7/9OAc)-α-(2→6)-D-Glc-α-(1→]. Like the serogroup W135 saccharide, it has variable O-acetylation at sialic acid 7 and 9 positions [84].

The *H.influenzae* type b capsular saccharide is a polymer of ribose, ribitol, and phosphate ['PRP', (poly-3-β-D-ribose-(1, 1)-D-ribitol-5-phosphate)].

In addition to being useful for analysing full-length capsular saccharides, the invention can be used with oligosaccharide fragments of them.

Other saccharides in conjugates can include glucans (*e.g.* fungal glucans, such as those in *Candida albicans*), and fungal capsular saccharides *e.g.* from the capsule of *Cryptococcus neoformans.* Other preferred conjugate saccharide antigens are eukaryotic saccharides *e.g.* fungal saccharides, plant saccharides, human saccharides (*e.g.* cancer antigens), *etc.* Other conjugate saccharides are lipopolysaccharides and lipooligosaccharides.

Conjugate saccharides that are charged (*e.g.* anionic) at neutral pH are preferred. Saccharides with multiple phosphate and/or multiple carboxylate groups can be analysed using the methods of the invention. The invention is thus particularly useful for analysing samples comprising polyanionic saccharides.

### Vaccines

Preferred samples used in the present invention are vaccines comprising conjugated saccharide.

Preferred conjugate vaccines comprise immunogens protecting against:
- *Haemophilus influenzae* type b (Hib);
- *Neisseria meningitidis* (meningococcus) of serogroups A, C W135 and/or Y;
- *Streptococcus pneumoniae* (pneumococcus);
- *Streptococcus agalactiae* (group B streptococcus);
- *Pseudomonas aeruginosa;* or
- *Staphylococcus aureus,*
either singly or in combination.

Preferred combination conjugate vaccines comprise:
- mixtures of conjugates from each of meningococcal serogroups C and Y;
- mixtures of conjugates from each of meningococcal serogroups C, W135 and Y;
- mixtures of conjugates from each of meningococcal serogroups A, C, W135 and Y;
- mixtures of conjugates from meningococcal serogroups A and C;
- mixtures of pneumoccal conjugates;
- mixed meningococcal and Hib conjugates (*e.g.* mixtures of Hib conjugates and conjugates from each of meningococcal serogroups A and C); or
- combined meningococcal, pneumococcal and Hib conjugates.

Vaccines comprising CRM-Hib (*i.e.* Hib saccharide conjugated to a CRM₁₉₇ carrier) and/or CRM-MenA are particularly preferred. Other preferred vaccines are those containing:
- a conjugate of diphtheria toxoid and a *N.meningitidis* serogroup A, C, W135 and/or Y saccharide;
- a conjugate of tetanus toxoid and Hib saccharide; or
- a conjugate of *H.influenzae* protein D and a *N.meningitidis* serogroup A, C, W135 and/or Y saccharide.

In addition to the conjugate, the vaccine may contain one or more of:
- a protein antigen from serogroup B of *N.meningitidis;*
- preparations of vesicles prepared from *N.meningitidis* serogroup B;
- an antigen from hepatitis A virus, such as inactivated virus [*e.g.* 36, 37];
- an antigen from hepatitis B virus, such as the surface and/or core antigens [*e.g.* 37, 38];
- an antigen from *Bordetella pertussis,* such as pertussis holotoxin (PT) and filamentous haemagglutinin (FHA) from *B.pertussis,* optionally also in combination with pertactin and/or agglutinogens 2 and 3. Cellular pertussis antigens may be used instead;
- a diphtheria antigen, such as a diphtheria toxoid [*e.g.* chapter 13 of ref. 1];
- a tetanus antigen, such as a tetanus toxoid [*e.g.* chapter 27 of ref. 1]; or
- polio antigen(s), *e.g.* IPV.

Such antigens may be adsorbed to an aluminium salt adjuvant (*e.g.* a hydroxide or a phosphate). Any further saccharide antigens are preferably included as conjugates.

### Solid Phase Extraction

It is not critical to the invention whether any non-saccharide components of the sample are retained with the conjugated saccharide or with the unconjugated saccharide. Provided the unconjugated saccharide and the conjugated saccharide end up separate from each other, then the unconjugated saccharide is considered to have been separated from the conjugated saccharide, regardless of which of the saccharide components is moved, which is present in the retentate or which is present in the effluent, and regardless whether either of the saccharide components is separated with other non-saccharide components.

The solid phase extraction device may be selected to retain either the conjugated saccharide or the unconjugated saccharide. When the conjugated saccharide is retained in the solid phase extraction device as a retentate, the unconjugated saccharide will be comprised in the effluent. Conversely, when the unconjugated saccharide is retained in the solid phase extraction device as retentate, the conjugated saccharide will be comprised in the effluent. It is preferred, however, that the conjugated saccharide is present in the retentate and the unconjugated saccharide is present in the effluent.

When the conjugated saccharide is present in the retentate, it is preferred that the solid phase extraction device is sufficiently efficient such that it retains at least 90% of the conjugated saccharide and allows at least 90% of the unconjugated saccharide to pass through as effluent. When the unconjugated saccharide is present in the retentate, it is preferred that the solid phase extraction device is sufficiently efficient such that it retains at least 90% of the unconjugated saccharide and allows at least 90% of the conjugated saccharide to pass through as effluent. In order to allow quantitative analysis of the sample when using solid phase extraction devices, it may be preferred to calibrate the solid phase extraction device by measuring the degree of separation of a standard sample containing known amounts of conjugated and unconjugated saccharide. Such calibration will become more desirable as the efficiency of retention diminishes.

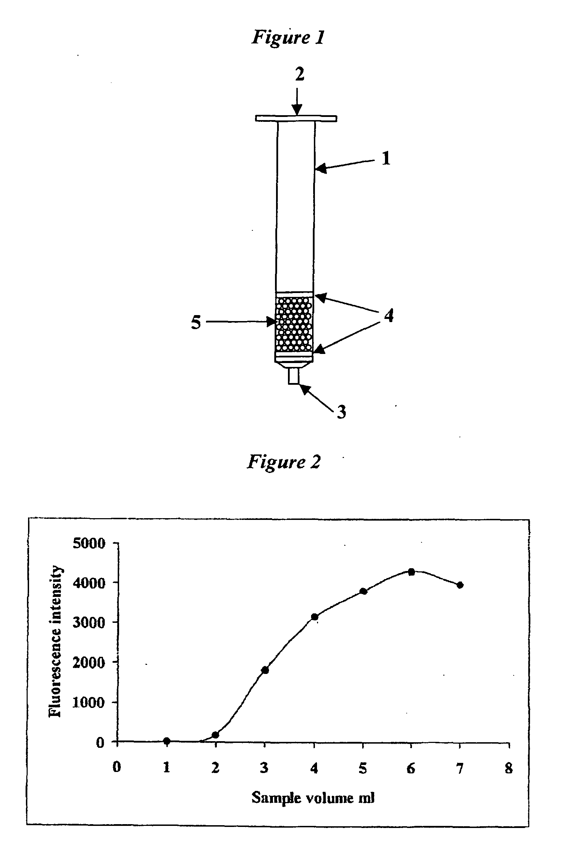

Solid phase extraction devices comprise a solid phase extraction sorbent and are well known in the art. Solid phase extraction devices are usually provided in the form of a tube or cartridge. A known device is shown in figure 1 and typically comprises a syringe barrel-like body 1 (usually of polypropylene) comprising an opening 2 into which the sample is added and an outlet 3 through which the effluent (and any subsequent eluate) passes. The body contains solid phase extraction sorbent 5 for retaining the target compound (the retentate) of the sorbent which is supported by frits 4.

Solid phase extraction may be used to separate the conjugated or unconjugated saccharide in three ways: (i) selective extraction; (ii) selective washing; or (iii) selective elution. It is preferred that the conjugated saccharide and unconjugated saccharide are separated by selective extraction and it is particularly preferred that the conjugated saccharide is retained as the retentate and the unconjugated saccharide passes through the SPE device as the effluent.

### Selective Extraction

Selective extraction involves using an SPE sorbent that will bind either the unconjugated saccharide or the conjugated saccharide. The sample is passed through the solid phase extraction device causing the unconjugated saccharide or conjugate saccharide, as appropriate, to be retained on the sorbate and allowing the other to pass through the SPE device as the effluent.

Depending on whether the saccharide content of the separated conjugated saccharide or the saccharide content of the separated unconjugated saccharide is of interest, the effluent may either be discarded or retained for saccharide analysis. Similarly, the solid phase extraction device may either be discarded or the absorbed saccharide retentate may be eluted for subsequent saccharide analysis.

A wide variety of different SPE sorbents are known to the skilled person along with procedures to select an appropriate SPE sorbent for separating given materials. Guidance for the selection of appropriate SPE devices in addition to appropriate solvents and conditions (such as pH) may be found in references 85 and 86.

In distinction to the unconjugated saccharides, the conjugated saccharides comprise a carrier and when the conjugated saccharide is to be retained in the sorbent, the sorbent is typically chosen to bind selectively to the carrier. The carriers are usually proteins and therefore sorbents for binding proteins are preferred and typically used in the reverse phase (polar liquid phase, nonpolar modified solid phase).

Such protein-binding sorbents include alkyl-bonded silica sorbents, *e.g.* C₁₈ (octadecyl), C₈ (octyl) or C₄ (butyl) bonded to silica, typically having the structure: 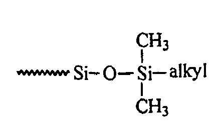 where the hydrophilic silanol groups at the surface of the raw silica packing have been chemically modified with hydrophobic alkyl. C₁₈ and C₄ alkyl-bonded silica sorbents are preferred, with C₄ being particularly preferred. The sorbents are typically sold pre-packed in the tube or cartridge. Especially preferred Isolute^{™} C₁₈ solid phase extraction cartridges (200mg) are available from International Sorbent Technology and Bio-Select^{™} C₁₈ and C₄ solid-phase extraction cartridges (50mg) are available from Vydac.

Other protein-binding sorbents include aryl-bonded silica sorbents, *e.g.* phenyl bonded to silica, typically having the structure: 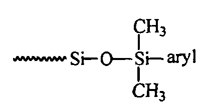 where the hydrophilic silanol groups at the surface of the raw silica packing have been chemically modified with hydrophobic aryl.

Bacterial capsular saccharides are typically anionic and when the unconjugated saccharide is to be retained in the sorbent, an ion exchange sorbent maybe used to bind selectively with the unconjugated saccharide. Such sorbents include aminopropyl bonded silica and quaternary amine bonded silica with Cl⁻ counterion. Since the ion exchange sorbents principally operate by electrostatic interaction, the conditions (*e.g.* solvent and pH), are adjusted to ensure the unconjugated saccharide and the sorbent are charged (negatively and positively, respectively) while the uncharged or positively charged conjugated saccharide passes through the sorbent in the effluent.

Once the appropriate SPE sorbent has been chosen, the SPE sorbent is usually conditioned by rinsing the tube with solvent before extracting the sample.

To enhance the retention of the appropriate saccharide, the pH, salt concentration and/or organic solvent content of the sample solution may be adjusted. The sample may also be pre-filtered to avoid clogging of the SPE device, *e.g.* to remove dust.

In order to ensure high separation efficiency, the sample should be passed slowly through the device, *e.g.* dropwise flow, and preferably under vacuum through the effluent outlet or under positive pressure through the SPE device opening.

For quantitative analysis, the amount of sample passed through the SPE device generally should not exceed its breakthrough volume, *i*.*e*. the point at which the sample volume exceeds the retention capacity of the sorbent. The breakthrough volume of a device for a given sample can be determined by plotting a breakthrough curve, in which a sample of fixed concentration and at a constant velocity enters the SPE device and the effluent analysed. The retentate is quantitatively retained during the initial phase by the sorbent until the moment in which the sample volume exceeds the retention capacity of the sorbent. The point on the curve at which some of the retentate is detected at the outlet of SPE device is the breakthrough volume.

After passing the sample through the SPE device, the sorbent is washed to remove unretained materials (*e.g.* unconjugated saccharide where the conjugated saccharide is the retentate). A typical polar solvent for washing is aqueous buffer. A typical non-polar solvent for washing is acetonitrile.

Finally, the compounds retained in the SPE sorbent may be eluted with an appropriate solvent. A typical polar solvent for eluting is aqueous buffer. A typical non-polar solvent for eluting is acetonitrile.

### Selective Washing/Selective Elution

Selective washing or selective elution involves using an SPE sorbent that will initially bind both the conjugated saccharide and the conjugated saccharide. The sample is passed through the solid phase extraction device causing both the conjugated and the unconjugated saccharide to be retained on the sorbate. Other materials may pass through the SPE device as the effluent.

The SPE sorbent is then washed with a wash solution (typically a solvent) which leaves either the unconjugated or conjugated saccharide bound to the SPE sorbent and elutes the other saccharide as an eluate. Depending on whether the saccharide content of the separated conjugated saccharide or the saccharide content of the separated unconjugated saccharide is of interest, the eluate may either be discarded or retained for saccharide analysis. Similarly, the solid phase extraction device may either be discarded or the absorbed saccharide retentate may be eluted for subsequent saccharide analysis.

A wide variety of different SPE sorbents are known to the skilled person along with procedures to select an appropriate SPE sorbent for separating given materials. Guidance for the selection of appropriate SPE devices in addition to appropriate solvents and conditions (such as pH) may be found in references 85 and 86.

The steps mentioned above in relation to selective extraction to ensure high separation efficiency and/or quantitative analysis may also be applied to selective washing/elution.

### General

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value *x* means, for example, *x*±10%.

### BRIEF DESCRIPTION OF DRAWINGS

Figure 1 shows a schematic diagram of a known solid phase extraction device.
Figure 2 shows a fluorescence spectra of collected aliquots of CRM-Hib glycoconjugate passed through a C₄ cartridge (wide pore) with 50 mg of sorbent to determine breakthrough volume.
Figure 3 shows a standard curve of fluorescence intensity versus protein concentration
Figure 4 shows a fluorescence spectra of collected aliquots of CRM-Hib glycoconjugate passed through a C₁₈ cartridge (wide pore) with 50 mg of sorbent to determine breakthrough volume.

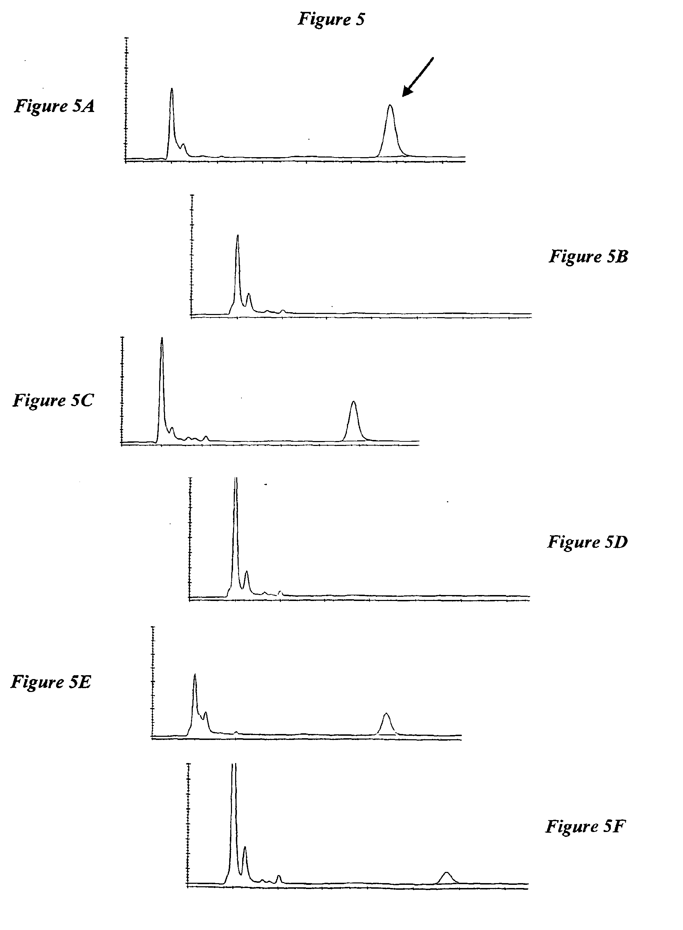

Figures 5A-5F show chromatograms of CRM-MenC alone and when admixed with CRM-MenA. In each figure, time is plotted on the *x* axis (0-15 minutes) and the output of the PAD is plotted on the *y* axis. The elution of sialic acid can be seen at the right hand peak indicated by the arrow in figure 5A. Figure 5A shows the chromatogram of CRM-MenC (total saccharide), figure 5B shows the chromatogram of CRM-MenC after SPE treatment with the C4 cartridge, figure 5C shows the chromatogram of CRM-MenA + CRM-MenC (total saccharide) after SPE treatment with the C4 cartridge, figure 5D shows the chromatogram of CRM-MenA + CRM-MenC (free saccharide) after SPE treatment with the C4 cartridge, figure 5E shows the chromatogram of 5% MenC oligosaccharide standard, and figure 5F shows the chromatogram of CRM-MenA + CRM-MenC + 5% MenC oligosaccharide (free saccharide) after SPE treatment with the C4 cartridge.

### MODES FOR CARRYING OUT THE INVENTION

### 1. CRM-Hib glycoconjugate vaccine

### 1.1 Samples and Reagents

Hib oligosaccharide and CRM-Hib (Hib oligosaccharide covalently attached to CRM₁₉₇) were produced by Chiron Vaccines (Siena, Italy). NaOH for chromatography were Sodium Hydroxide 1 N (C.Erba, Milan, Italy). Ribitol standard was of purity ≥99% (HPLC) (Fluka, Switzerland). Trifluoroacetic acid was of purity ≥99.5% (T) (Fluka, Switzerland). Isolute^{™} C18 solid-phase extraction cartridges (200 mg) were purchased from International Sorbent Technology (Mid Glamorgan, UK); Bio-Select^{™} C18 and C4 solid-phase extraction cartridges (50 mg) were purchased from Vydac.

### 1.2 Quantitative determination of saccharide in CRM-Hib glycoconjugate vaccines

CRM-Hib standards (1.0 mL with a saccharide concentration range of 0.18-1.8 µg/mL) and ribitol standard (0.075-0.75 µg/ml) were treated with 50 µl of hydrochloric acid (HCl) 6 M (final HCl concentration: 0.3 M); samples were heated at 100°C for 2 hours in a closed screw-cap test tube to hydrolyse the saccharides. Hydrolysates were centrifuged after incubation to combine the condensate with the bulk liquid. The hydrolysates were then neutralised with 400 µl of NaOH 1 M and filtered (0.22 µm). Analysis of the hydrolysed products was performed using a Dionex DX-500 chromatography system fitted with a GP40 pump, ED40 detector and AS3500 autosampler. The system was equipped with a CarboPac MA1 column (4 x 250 mm) in combination with a CarboPac MA1 guard column and operated at room temperature. Separation was performed with a flow rate of 0.4 mL/min using an isocratic elution with NaOH 580 mM. The effluent was monitored using a pulsed electrochemical detector (ED40) in the pulsed amperometric mode with a gold working electrode and an Ag/AgCl reference electrode. A quadruple-potential waveform was applied using the following settings: E1=0.1 V, tl=400 ms; E2=-2.0 V, t2=20 ms; E3=0.6 V, t3=10 ms; E4=-0.1 V, t4=60 ms. Integration occurs from 200 ms to 400 ms during E1 application. The resulting chromatographic data were integrated and processed using PeakNet data reduction software (Dionex).

### 1.3 SPE for CRM-Hib glycoconjugate vaccines

The CRM-Hib solution (20 µg/ml total saccharide measured in *1.2* above) was applied onto a solid-phase extraction cartridge previously pre-conditioned by washing sequentially with methanol (1 column volume), water (3 column volume) and NaCl 0.9% (1 column volume). The sample solution was allowed to run through by gravity and the cartridge was washed with 1 ml of NaCl 0.9%. The eluent was collected in a volumetric flask (2 ml) and an aliquot was analysed by ribitol assay. When the vaccine was formulated with aluminium-containing adjuvants, SPE was applied to the supernatant after centrifugation of the suspension.

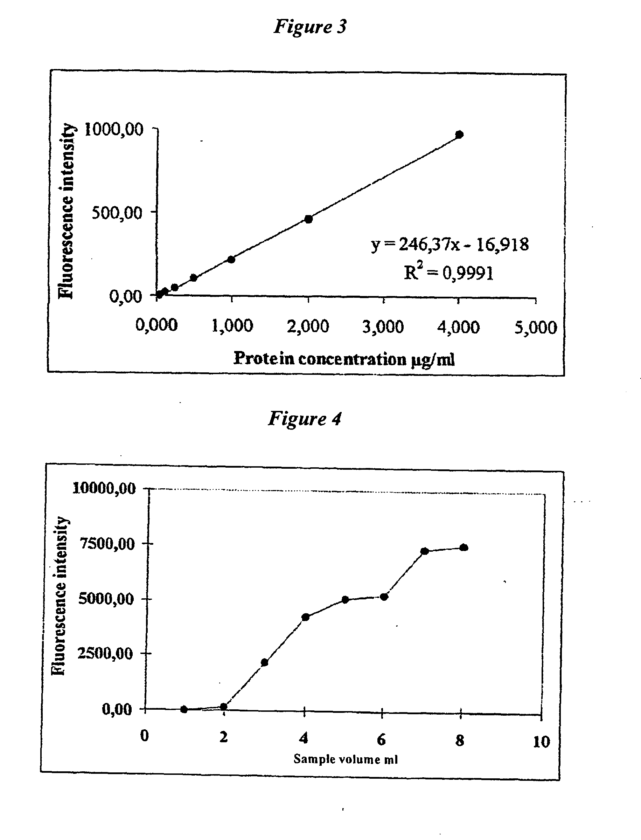

The breakthrough volume for the SPE of CRM-Hib glycoconjugate was also determined. Experiments were performed with a solution of CRM-Hib with a saccharide concentration of 20 µg/ml (typical concentration of a vaccine dose). A C₄ cartridge (wide pore) with 50 mg of sorbent which retains the conjugated saccharide and allows unconjugated saccharide to pass through as the effluent was used. The CRM-Hib solution was applied onto the pre-conditioned cartridge and it was allowed to run through by gravity as previously described. Data obtained from off-line detection of collected effluent aliquots (fluorescence spectroscopy detection) are shown in Figure 2. The aliquots 1 and 2 were also analysed for protein concentration with a quantitative fluorescence method using CRM-Hib solution to obtain a standard curve (Figure 3). It has been calculated that a level lower than 2.5% of initial protein concentration was found in the aliquot 2 and consequently that a breakthrough volume for a CRM-Hib solution was 2 ml. A similar experiment was performed with a C₁₈ cartridge (wide pore) with 50 mg of sorbent which retains the conjugated saccharide and allows unconjugated saccharide to pass through as the effluent. Results obtained in this experiment were shown in Figure 4. It can be concluded that the C₁₈ cartridge had the same breakthrough volume previously determined for the C₄ cartridge.

In order to check the possibility of binding conjugated vaccine to SPE sorbent while unconjugated saccharide passes through, some experiments with CRM-Hib alone and CRM-Hib spiked with oligo-Hib standards were performed. In these experiments saccharide quantification was made by use of the ribitol assay previously described. Results displayed in Table 1 show that a better recovery of free saccharide was obtained with a cartridge with 50 mg of sorbent. Moreover, best results were obtained with a C₄ cartridge, which shows an optimal range of recoveries in the three different concentration levels of spike solution (95-105%).

**Table 1**

| | C₁₈ (200 mg) | |
|---|---|---|
| Sample | Free saccharide µg/ml | Recovery % |
| CRM-Hib | 0.37 | |
| CRM-Hib + oligo-Hib (+0.2 µg/ml) | 0.37 | 0 |
| CRM-Hib + oligo-Hib (+1.0 µg/ml) | 1.16 | 79 |
| CRM-Hib + oligo-Hib (+2.0 µg/ml) | 2.15 | 89 |
| | | |

| | C₁₈ (50 mg - wide pore) | |
|---|---|---|
| Sample | Free saccharide µg/ml | Recovery % |
| CRM-Hib | 0.10 | |
| CRM-Hib + oligo-Hib (+0.2 µg/ml) | 0.24 | 70 |
| CRM-Hib + oligo-Hib (+1.0 µg/ml) | 1.16 | 106 |
| CRM-Hib + oligo-Hib (+2.0 µg/ml) | 1.96 | 93 |
| | | |

| | C₄ (50 mg - wide pore) | |
|---|---|---|
| Sample | Free saccharide µg/ml | Recovery % |
| CRM-Hib | 0.22 | |
| CRM-Hib + oligo-Hib (+0.2 µg/ml) | 0.41 | 95 |
| CRM-Hib + oligo-Hib (+1.0 µg/ml) | 1.28 | 106 |
| CRM-Hib + oligo-Hib (+2.0 µg/ml) | 2.32 | 105 |

### 2. DTP/CRM-Hib glycoconjugate vaccines

### 2.1. SPE for DTwP/CRM-Hib glycoconjugate vaccines

A DTP/CRM-Hib glycoconjugate was analysed in the same way as the CRM-Hib glycoconjugate in example 1, except a 10 mM phosphate buffer was added to extract the Hib conjugate into the supernatant before centrifugation.

The possibility of binding conjugated vaccine from a combination vaccine to SPE sorbent while unconjugated saccharide passes through was also investigated with DTP/CRM-Hib alone and DTP/CRM-Hib spiked with oligo-Hib samples. Saccharide quantification was made by use of the ribitol assay previously described. Results displayed in Table 2 show that a better recovery of free saccharide was obtained with a cartridge with 50 mg of sorbent.

**Table 2**

| | C₄ (50mg - wide pore) water washing | | C₄ (50mg - wide pore) saline washing | |
|---|---|---|---|---|
| Sample | Free saccharide µg/ml | Recovery % | Free saccharide µg/ml | Recovery % |
| DTP/CRM-Hib | 2.3 | | | |
| DTP/CRM-Hib + oligo-Hib (+1.0 µg/ml) | 3.9 | 118 | | |
| DTP/CRM-Hib + oligo-Hib (+2.0 µg/ml) | 4.7 | 109 | 2.2 | 110 |
| | | | | |

| | C₄ (100mg - wide pore) water washing | | C₄ (100mg - wide pore) saline washing | |
|---|---|---|---|---|
| Sample | Free saccharide µg_{/}ml | Recovery % | Free saccharide µg/ml | Recovery % |
| DTP/CRM-Hib | 2.0 | | | |
| DTP/CRM-Hib + oligo-Hib (+1.0 µg/ml) | 2.7 | 90 | | |
| DTP/CRM-Hib + oligo-Hib (+2.0 µg/ml) | 3.4 | 87 | 2.0 | 100 |

It can therefore be seen that the invention advantageously allows the determination of the unconjugated saccharide content even in the presence of a DTwP vaccine.

### 3. CRM-MenA glycoconjugate vaccine

### 3.1 Samples and Reagents

MenA polysaccharide and CRM-MenA were experimental products prepared by Chiron Vaccines (Siena, Italy). NaOH for chromatography were Sodium Hydroxide 1 N (C.Erba, Milan, Italy). Trifluoroacetic acid was of purity ≥99.5% (T) (Fluka, Switzerland). Isolute^{™} C18 solid-phase extraction cartridges (200 mg) were purchased from International Sorbent Technology (Mid Glamorgan, UK); Bio-Select^{™} C18 and C4 solid-phase extraction cartridges (50 mg) were purchased from Vydac.

### 3.2 Quantitative determination of saccharide in CRM-MenA glycoconjugate vaccines

CRM-MenA samples and MenA polysaccharide standard (ranging between 1 and 8 µg/ml of saccharide) were treated with 150 µl of trifluoroacetic acid (TFA) 8 M (final TFA concentration: 2 M) and then heated at 100°C for 2 h in a closed screw-cap test tube to hydrolyse the saccharides. Hydrolysates were centrifuged after incubation to combine the condensate with the bulk liquid. To neutralise the hydrolysates 600 µl of NaOH 2 M were added. All samples were filtered (0.22 µm) and transferred to autosampler vials. Analysis of the hydrolysed products was performed using a Dionex DX-500 chromatography system fitted with a GP40 pump, ED40 detector and AS3500 autosampler. The system was equipped with a CarboPac PA1 column (4 x 250 mm) in combination with a CarboPac PA1 guard column and operated at room temperature. Separation was performed with a flow rate of 1 ml/min using a gradient elution as follows: eluent A was NaOH 100 mM and eluent B was NaOH 100 mM containing 1 M sodium acetate; a gradient from 20%B to 50%B over 15 minutes was applied. The effluent was monitored using an electrochemical detector (ED40) in the pulsed amperometric mode with a gold working electrode and an Ag/AgCl reference electrode. A quadruple-potential waveform was applied using the following settings: E1 = 0.1 V, t1 = 400 ms; E2 = -2.0 V, t2 = 20 ms; E3 = 0.6 V, t3 = 10 ms; E4 = -0.1 V, t4 = 60 ms. Integration occurs from 200 to 400 ms during E1 application. The resulting chromatographic data were integrated and processed using PeakNet data reduction software (Dionex).

### 3.2. SPE for CRM-MenA glycoconjugate vaccines

Lyophilized CRM-MenA solution and bulk CRM-MenA solution without excipients (each 20 µg/ml total saccharide measured in *3.1* above) were analysed. Each sample was applied onto a solid-phase C₄ extraction cartridge previously pre-conditioned by washing sequentially with methanol (1 column volume), water (3 column volume) and phosphate buffer 5 mM + 0.9% NaCl pH 7.2 (1 column volume). The sample solution was allowed to run through by gravity and the cartridge was washed with 1 ml of acetonitrile 10%/aqueous TFA 0.05%. The eluent was collected in a volumetric flask (2 ml) and an aliquot was analysed by mannosamine-6-P assay.

Similar experiments with CRM-MenA spiked with ∼2 µg/ml MenA standard were also performed.

The results are shown in table 3.

**Table 3**

| Sample | Free saccharide | Recovery % |
|---|---|---|
| Lypophilized CRM-MenA | 3.6 | |
| Lypophilized CRM-MenA + standard (+1.97 µg/ml) | 5.4 | 91.4 |
| | | |

| Sample | Free saccharide | Recovery % |
|---|---|---|
| Bulk CRM-MenA | 1.8 | |
| Bulk CRM-MenA + standard (+2.12 µg/ml) | 4.0 | 103.8 |

### 4. CRM-MenA + CRM-MenC glycoconjugate vaccine

### 4.1 Samples and Reagents

CRM-MenC was concentrated bulk diluted to 20 µg/ml of saccharide in 5mM phosphate buffer pH 7.2 with 0.9% NaCl. CRM-MenA was concentrated bulk diluted to 20 µg/ml of saccharide in 5mM phosphate buffer pH 7.2 with 0.9% NaCl. CRM-MenA + CRM-MenC was diluted to 20 µg/ml of saccharide in 5mM phosphate buffer pH 7.2 with 0.9% NaCl.

### 4.2 Quantitative determination of MenC oligosaccharide in CRM-MenA + CRM-MenC glycoconjugate vaccines

The samples were diluted in 5mM NaPi pH 7.2 + 0.9% NaCl and treated with acid (HCl 0.1 M) for 3 h at 80°C to hydrolyse the saccharides. To neutralise the hydrolysates, NaOH was added stoichiometrically. The resulting hydrolysed products were separated using HPAEC-PAD by 15 minutes isocratic elution with 50 mM sodium acetate in 100mH NaOH using a CarboPac PA1 column equipped with PA1 guard at a flow rate of 0.8 ml/min with 50 µl sample injection. Quantification of sialic acid was carried out with PAD detection with a triple-potential waveform for carbohydrates. Calibration was carried out with a sialic acid range between 5.00 and 0.63 µg/ml for total saccharide quantification.

### 4.3 SPE for CRM-MenA + CRM-MenC glycoconjugate vaccines

The CRM-MenA + CRM-MenC solution (measured in 4.2 above) was applied onto a wide pore C4 solid-phase extraction cartridge previously pre-conditioned by washing sequentially with methanol (3 ml), water (6 ml) and physiological saline (4 ml). 1.0 ml of sample solution was allowed to run through by gravity and the cartridge was washed with 1 ml of physiological saline by gravity. The eluate was collected in volumetric flasks (2 ml) and, where necessary, diluted to volume with a buffer solution containing 5mN NaPi pH 7.2 + 0.9% NaCl. The eluate was hydrolysed, separated and quantified for sialic acid as mentioned in *4.2* above, but with calibration of PAD detection with a sialic acid range between 0.31 and 0.04 µg/ml.

Since MenC saccharide is a α-(2→9)-linked homopolymer of sialic acid whereas MenA saccharide is a α-(1→6)-linked N-acetyl-mannosamine-6-phosphate homopolymer, sialic acid and mannosamine-6-phosphate monomers are obtained after hydrolysis. These molecules have different retention properties and therefore do not interfere when present in the same mixture, as shown in the chromatograms in figure 5.

The efficacy of the free MenC saccharide recovery was analysed by adding free MenC oligosaccharide to the CRM-MenA + CRM-MenC samples corresponding to 5% of the CRM-MenC glycoconjugate. The recovery is calculated by comparison to a glycoconjugate sample without additions. The results are shown in table 4.

**Table 4**

| Sample | A | B | C | D | E |
|---|---|---|---|---|---|
| | µg/ml CRM-MenC total | g/ml MenC not conjugated | µg/ml MenC OS added | % recovery addition (B/C x 100) | % free saccharide (B/A x 100) |
| CRM-MenC (20µg/ml) | 17.06 | <0.1 | 0.00 | n.d | <0.5* |
| CRM-MenA + CRM-MenC (20µg/ml) | 17.16 | <0.1 | 0.00 | n.d | <0.5* |
| CRM-MenA+ CRM-MenC (20µg/ml) + 1µg/ml OS-MenC | 16.78 | 0.99 | 1.00 | 98.48 | 5.88 |
| Mix OS-MenC 1 µg/ml | 1.00 | n.d. | n.d. | n.d. | n.d. |
| *peak area was smaller than that of the least concentrated standard (0.04µg/ml). therefore. being the sample dilution factor equal to 2.5. the percentage was calculated as follows: 0.04×2.5/20×100 | | | | | |

The recovery of the added oligosaccharides is close to 100% also in presence of CRM-MenA.

### 5. CRM-MenC + CRM-MenW+ CRM-MenYglycoconjugate vaccine

### 5.1 Samples and Reagents

The CRM-MenC + CRM-MenW + CRM-MenY glycoconjugate vaccine samples were supernatants of MenCWY vaccine formulated with aluminium phosphate adjuvant, with Al³⁺ concentration of 0.6 mg/ml, in 10 mM phosphate buffer pH 7.2 containing 0.005% Tween 80 and 0.9% NaCl.

### 5.2 Quantitative determination of MenC oligosaccharide in CRM-MenC + CRM-MenW + CRM-MenYglycoconjugate vaccines

The samples were diluted in milliQ water and treated with acid (1M TFA) for 2 h at 90°C to hydrolyse the saccharides. To neutralise the hydrolysates, NaOH was added stoichiometrically. The resulting hydrolysed products were separated using HPAEC-PAD by gradient elution with 20 minute steps (5 min with 25mM sodium acetate with 100mM NaOH, 5 min of a 25mM to 100mM sodium acetate gradient with 100mM NaOH, 5 min with 100mM sodium acetate with 100mM NaOH, and a final system reconditioning of 5 min) using a CarboPac PA1 column equipped with PA1 guard at a flow rate of 1 ml/min with 50 µl sample injection. Quantification of sialic acid was carried out with PAD detection with a triple-potential waveform for carbohydrates. Calibration was carried out with MenC polysaccharide, MenY polysaccharide and MenW polysaccharide ranging from 0.2 to 4.0 µg/ml each, equivalent to glucose+galactose 0.8-16.8 nmol/ml (0.1-3.0µg/ml) and sialic acid 1.5-29.8 nmol/ml (0.5-9.2µg/ml).

### 5.3 SPE for CRM-MenC + CRM-MenW + CRM-MenY glycoconjugate vaccines

The CRM-MenC + CRM-MenW + CRM-MenY solution (measured in 5.2 above) was applied onto a wide pore C4 solid-phase extraction cartridge previously pre-conditioned by washing sequentially with methanol (3 ml), water (6 ml) and physiological saline (4 ml). 1.0 ml of sample solution was allowed to run through by gravity and the cartridge was washed with 1 ml of physiological saline by gravity. The eluate was collected in a volumetric flasks (2 ml) and, where necessary, diluted to volume with a buffer solution containing 5mN NaPi pH 7.2 + 0.9% NaCl. The eluate was hydrolysed, separated and quantified for sialic acid as mentioned in 5.2 above.

The efficacy of the free saccharide recovery was analysed by adding free MenC oligosaccharide to the CRM-MenC + CRM-MenW + CRM-MenY samples. The recovery is calculated by comparison to a glycoconjugate sample without additions. The results are shown in table 5.

**Table 5**

| Sample | A | B | C | D | E |
|---|---|---|---|---|---|
| | µg/ml MenC-CRM | µg/ml MenC not conjugated | µg/ml MenC OS added | % recovery addition (B/C x 100) | % free saccharide (B/A x 100) |
| MenCWY-CRM (20µg/ml) | 21.11 | <1.0 | 0.00 | n.d | <5.0* |
| MenCWY-CRM (20µg/ml) + 2µg/ml OS-MenCWY | 30.10 | 3.39 | 3.50 | 96.84 | 11.27 |
| Mix OS-MenCWY 2µg/ml | 3.50 | 2.93 | 3.50 | 83.51 | 83.51 |
| MenCWY-CRM (5µg/ml) | 4.77 | <1.0 | 0.00 | n.d | <20.0* |
| MenCYN-CRM (5µg/ml) +1.5µg/ml OS-MenCWY | 5.84 | 1.14 | 1.04 | 109.21 | 19.47 |
| Mix OS-MenCWY 1.5µg/ml | 1.04 | 1.23 | 1.04 | 118.01 | 118.01 |
| *LOQ of the method | | | | | |

The recovery of the added oligosaccharides is dose to 100% also in presence of CRM-MenW and CRM-MenY. Moreover, the adjuvant does not interfere with saccharide recovery.

### REFERENCES (the contents of which are hereby incorporated by reference)

[1] Vaccines (eds. Plotkin et al.) 4th edition, ISBN: 0721696880.
[2] Baker et al. (2003) J Infect Dis 188:66-73.
[3] Theilacker et al. (2003) Infect Immun 71:3875-84.
[4] Anonymous (2003) Drugs R D 4:383-5.
[5] Jones (2001) Curr Opin Investig Drugs 2:47-49.
[6] WO02/058737.
[7] WO03/007985.
[8] Rennels et al. (2002) Pediatr Infect Dis J 21:978-979.
[9] Campbell et al. (2002) J Infect Dis 186:1848-1851.
[10] Costantino et al. (1992) Vaccine 10:691-698.
[11] Lieberman et al. (1996) JAMA 275:1499-1503.
[12] Darkes & Plosker (2002) Paediatr Drugs 4:609-630.
[13] Ugozzoli (2002) J Infect Dis 186:1358-61.
[14] Granoff et al. (1997) Infect Immun 65:1710-5.
[15] Paradiso & Lindberg (1996) Dev Biol Stand 87:269-275.
[16] Corbel (1996) Dev Biol Stand 87:113-124.
[17] WO03/080678.
[18] Klug (1996) Dev Biol Stand 87:263-267.
[19] Plumb & Yost (1996) Vaccine 14:399-404.
[20] Jumel et al. (2002) Biotechnol Appl Biochem 36:219-226.
[21] Bardotti et al. (2000) Vaccine 18:1982-1993
[22] UK patent application 0406013.3
[23] Hardy et al. (1988) Anal Biochem 170:54-62.
[24] Wang et al. (1990) Anal Biochem 190:182-187.
[25] WO02/09643.
[26] Katial et al. (2002) Infect Immun 70:702-707.
[27] WO01/52885.
[28] European patent 0301992.
[29] Bjune et al. (1991) Lancet 338(8775):1093-1096.
[30] Fukasawa et al. (1999) Vaccine 17:2951-2958.
[31] WO02/09746.
[32] Rosenqvist et al. (1998) Dev. Biol. Stand. 92:323-333.
[33] Watson (2000) Pediatr Infect Dis J 19:331-332.
[34] Rubin (2000) Pediatr Clin North Am 47:269-285, v.
[35] Jedrzejas (2001) Microbiol Mol Biol Rev 65:187-207.
[36] Bell (2000) Pediatr Infect Dis J 19:1187-1188.
[37] Iwarson (1995) APMIS 103:321-326.
[38] Gerlich et al. (1990) Vaccine 8 Suppl:S63-68 & 79-80.
[39] Gustafsson et al. (1996) N. Engl. J. Med 334:349-355.
[40] Rappuoli et al. (1991) TIBTECH 9:232-238.
[41] Del Guidice et al. (1998) Molecular Aspects of Medicine 19:1-70.
[42] Sutter et al. (2000) Pediatr Clin North Am 47:287-308.
[43] Zimmerman & Spann (1999) Am Fam Physician 59:113-118, 125-126.
[44] Ramsay et al. (2001) Lancet 357(9251):195-196.
[45] Lindberg (1999) Vaccine 17 Suppl 2:S28-36.
[46] Buttery & Moxon (2000) J R Coll Physicians Lond 34:163-168.
[47] Ahmad & Chapnick (1999) Infect Dis Clin North Am 13:113-133, vii.
[48] Goldblatt (1998) J. Med. Microbiol. 47:563-567.
[49] European patent 0477508.
[50] US patent 5,306,492.
[51] WO98/42721.
[52] Conjugate Vaccines (eds. Cruse et al.) ISBN 3805549326, particularly vol. 10:48-114.
[53] Hermanson (1996) Bioconjugate Techniques ISBN: 0123423368 or 012342335X.
[54] US patent 4,761,283
[55] US patent 4,356,170
[56] WO00/10599
[57] Gever et al. Med. Microbiol. Immunol, 165 : 171-288 (1979).
[58] US patent 4,057,685.
[59] US patents 4,673,574; 4,761,283; 4,808,700.
[60] US patent 4,459,286.
[61] US patent 4,965,338
[62] US patent 4,663,160.
[63] Anonymous (Jan 2002) *Research Disclosure,* 453077.
[64] Anderson (1983) Infect Immun 39(1):233-238.
[65] Anderson et al. (1985) J Clin Invest 76(1):52-59.
[66] EP-A-0372501.
[67] EP-A-0378881.
[68] EP-A-0427347.
[69] WO93/17712
[70] WO94/03208.
[71] WO98/58668.
[72] EP-A-0471177.
[73] WO91/01146
[74] Falugi et al. (2001) Eur Immunol 31:3816-3824.
[75] Baraldo et al, (2004) Infect Immun. 72:4884-7
[76] EP-A-0594610.
[77] WO00/56360.
[78] WO02/091998.
[79] WO01/72337
[80] WO00/61761.
[81] WO99/42130
[82] Glode et al. (1979) J Infect Dis 139:52-56
[83] WO94/05325; US patent 5,425,946.
[84] United Kingdom patent application 0323103.2.
[85] Supelco Guide to Solid Phase Extraction, Bulletin 910 (1998)
[86] J. Chrom. A, 856 (1999): 3-54

## Claims

1. A method of analysing a sample's unconjugated saccharide content, comprising the steps of (i) passing the sample through a solid phase; extraction device to separate conjugated saccharide from unconjugated saccharide and obtain a specimen comprising separated unconjugated saccharide and (ii) analysing the specimen's saccharide content to give the unconjugated saccharide content of the sample.

2. A method of preparing a sample for analysis of ils unconjugated saccharide content, comprising the step of passing the sample through a solid phase extraction device to separate conjugated saccharide from unconjugated saccharide.

3. The method of claim 1 or 2 comprising the step of measuring the sample's total saccharide content.

4. The method of any of claims 1 to 3 wherein the conjugated Saccharide is a saccharide antigen conjugate to a carrier proteins.

5. The method of any of claims 1 to 4 wherein the sample is a vaccine.

6. The method of claim 5 wherein the vaccine is a glycoconjugate vaccine.

7. The method of claim 6 wherein the glycoconjugate vaccine is a single vaccine.

8. The method of claim 6 wherein the glycoconjugate vaccine is a combined vaccine.

9. The method of claim 8 wherein the combined glycoconjugate vaccine comprises a conjugate from meningoccocal serogroup C.

10. The method of claim 9 wherein the glycoconjugate vaccine comprises mixtures of conjugates from cach of meningococcal serogroups C and Y.

11. The method of claim 9 wherein the glycoconjugate vaccine comprises mixtures of conjugates from each of meningococcal serogroups C, W135 and Y.

12. The method of claim 9 wherein the glycoconjugate vaccine comprises mixtures of conjugates from each of meningococcal serogroups A, C, W 135 and Y.

13. The method of claim 9 wherein the glycoconjugate vaccine comprises mixtures of conjugates from each of meningococcal serogroups A and C.

14. The method of any of claims 9 to 13 comprising the step of analysing the sample's unconjugated content of *N.meningitidis* serogroup C saccharide.

15. A method of separating a conjugated saccharide component in a sample from an unconjugated saccharide component in the sample, comprising the step of passing the sample through a solid phase extraction device.

16. The use of a solid phase extraction device for separating a conjugated saccharide component in a sample from an unconjugated saccharide component in the sample.

17. The method of claim 15 or the use of claim 16 wherein the conjugated saccharide is a saccharide antigen conjugated to a carrier protein.

18. The method of any of claims 15 or 17 or the use of claim 16 or claim 17 wherein the sample is a vaccine.

19. The method or use of claim 18 wherein the vaccine is a glycoconjugate vaccine.

20. The method or use of claim 19 wherein the glycoconjugate vaccine is a single vaccine.

21. The method or use of claim 20 wherein the glycoconjugate vaccine is a combined vaccine.

22. The method or use of claim 21 wherein the combined glycoconjugate vaccine comprises a conjugate from meningoccocal serogroup C.

23. The method or use of claim 22 wherein the glycoconjugate vaccine comprises mixtures of conjugates from each of meningococcal serogroups C and Y.

24. The method or use of claim 22 wherein the glycoconjugate vaccine comprises mixtures of conjugates from each of meningococcal serogroups C, W135 and Y.

25. The method or use of claim 22 wherein the glycoconjugate vaccine comprises mixtures of conjugates from each of meningococcal serogroups A, C, W135 and Y.

26. The method or use of claim 22 wherein the glycoconjugate vaccine comprises mixtures of conjugates from each of meningococcal serogroups A and C.

27. A method of releasing a vaccine for use by physicians, comprising the steps of (a) manufacturing a vaccine comprising a conjugated saccharide; (b) analysing the vaccine's unconjugated saccharide content by a method of claim 1; and, if the results from step (b) indicate a saccharide content acceptable for clinical use, (c) releasing the vaccine for use by physicians.

28. A method for preparing a vaccine composition, comprising a step of analysing the vaccine's unconjugated saccharide content by a method of claim 1, including a step of pH measurement, followed by a step of adjusting the pH of the composition to a desired value *e.g*. between 6 and 8, or about 7.

29. A method for packaging a vaccine, comprising the steps of: (a) manufacturing a bulk vaccine containing a conjugated saccharide; (h) analysing the unconjugated saccharide content in the bulk vaccine by a method of claim 1; (e) optionally, analysing the hulk vaccine for pH and/or other properties; and, if the results from step (b) and (c) indicate that the bulk vaccine is acceptable for clinical use, (d) preparing and packaging the vaccine for human use from the bulk.

## Patentansprüche

1. Verfahren zur Analyse des Gehalts einer Probe an unkonjugiertem Saccharid, bei dem man: (i) die Probe durch eine Festphasen-Extraktionsvorrichtung leitet, um konjugiertes Saccharid von unkonjugiertem Saccharid zu trennen und eine Probe zu erhalten, die abgetrenntes unkonjugiertes Saccharid enthält, und (ii) den Saccharid-Gehalt der Probe analysiert, um den Gehalt der Probe an unkonjugiertem Saccharid zu erhalten.

2. Verfahren zur Herstellung einer Probe für die Analyse ihres Gehalts an unkonjugiertem Saccharid, bei dem man die Probe durch eine Festphasen-Extraktionsvorrichtung leitet, um konjugiertes Saccharid von unkonjugiertem Saccharid zu trennen.

3. Verfahren nach Anspruch 1 oder 2, bei dem man den Gesamtgehalt der Probe an Saccharid misst.

4. Verfahren nach einem der Ansprüche 1-3, wobei das konjugierte Saccharid ein Saccharid-Antigen ist, das an ein Trägerprotein konjugiert ist.

5. Verfahren nach einem der Ansprüche 1-4, wobei die Probe ein Impfstoff ist.

6. Verfahren nach Anspruch 5, wobei der Impfstoff ein Glycokonjugat-Impfstoff ist.

7. Verfahren nach Anspruch 6, wobei der Glycokonjugat-Impfstoff ein einzelner Impfstoff ist.

8. Verfahren nach Anspruch 6, wobei der Glycokonjugat-Impfstoff ein kombinierter Impfstoff ist.

9. Verfahren nach Anspruch 8, wobei der kombinierte Glycokonjugat-Impfstoff ein Konjugat von Meningokokken-Serogruppe C umfasst.

10. Verfahren nach Anspruch 9, wobei der Glycokonjugat-Impfstoff eine Mischung von Konjugaten von jeder der Meningokokken-Serogruppen C und Y umfasst.

11. Verfahren nach Anspruch 9, wobei der Glycokonjugat-Impfstoff eine Mischung von Konjugaten von jeder der Meningokokken-Serogruppen C, W135 und Y umfasst.

12. Verfahren nach Anspruch 9, wobei der Glycokonjugat-Impfstoff eine Mischung von Konjugaten von jeder der Meningokokken-Serogruppen A, C, W135 und Y umfasst.

13. Verfahren nach Anspruch 9, wobei der Glycokonjugat-Impfstoff eine Mischung von Konjugaten von jeder der Meningokokken-Serogruppen A und C umfasst.

14. Verfahren nach einem der Ansprüche 9-13, bei dem man den Gehalt der Probe an unkonjugiertem Saccharid von *N. meningitidis* der Serogruppe C analysiert.

15. Verfahren zur Trennung eines konjugierten Saccharid-Bestandteils in einer Probe von einem unkonjugierten Saccharid-Bestandteil in der Probe, bei dem man die Probe durch eine Festphasen-Extraktionsvorrichtung leitet.

16. Verwendung einer Festphasen-Extraktionsvorrichtung zur Trennung eines konjugierten Saccharid-Bestandteils in einer Probe von einem unkonjugierten Saccharid-Bestandteil in der Probe.

17. Verfahren nach Anspruch 15 oder Verwendung nach Anspruch 16, wobei das konjugierte Saccharid ein Saccharid-Antigen ist, welches an ein Trägerprotein konjugiert ist.

18. Verfahren nach einem der Ansprüche 15 oder 17 oder Verwendung nach einem der Ansprüche 16 oder 17, wobei die Probe ein Impfstoff ist.

19. Verfahren oder Verwendung nach Anspruch 18, wobei der Impfstoff ein Glycokonjugat-Impfstoff ist.

20. Verfahren oder Verwendung nach Anspruch 19, wobei der Glycokonjugat-Impfstoff ein einzelner Impfstoff ist.

21. Verfahren oder Verwendung nach Anspruch 20, wobei der Glycokonjugat-Impfstoff ein kombinierter Impfstoff ist.

22. Verfahren oder Verwendung nach Anspruch 21, wobei der kombinierte Glycokonjugat-Impfstoff ein Konjugat von Meningokokken-Serogruppe C umfasst.

23. Verfahren oder Verwendung nach Anspruch 22, wobei der Glycokonjugat-Impfstoff eine Mischung von Konjugaten von jeder der Meningokokken-Serogruppen C und Y umfasst.

24. Verfahren oder Verwendung nach Anspruch 22, wobei der Glycokonjugat-Impfstoff eine Mischung von Konjugaten von jeder der Meningokokken-Serogruppen C, W135 und Y umfasst.

25. Verfahren oder Verwendung nach Anspruch 22, wobei der Glycokonjugat-Impfstoff eine Mischung von Konjugaten von jeder der Meningokokken-Serogruppen A, C, W135 und Y umfasst.

26. Verfahren oder Verwendung nach Anspruch 22, wobei der Glycokonjugat-Impfstoff eine Mischung von Konjugaten von jeder der Meningokokken-Serogruppen A und C umfasst.

27. Verfahren zur Freigabe eines Impfstoffs für die Verwendung durch Ärzte, bei dem man (a) einen Impfstoff herstellt, der ein konjugiertes Saccharid umfasst; (b) den Gehalt des Impfstoffs an unkonjugiertem Saccharid durch ein Verfahren nach Anspruch 1 analysiert; und, wenn die Ergebnisse von Schritt (b) auf einen Saccharid-Gehalt verweisen, der für die klinische Verwendung akzeptabel ist, (c) die Freigabe des Impfstoffs für die Verwendung durch Ärzte.

28. Verfahren zur Herstellung einer Impfstoff-Zusammensetzung, bei dem man den Gehalt des Impfstoffs an unkonjugiertem Saccharid durch ein Verfahren nach Anspruch 1 analysiert, einschließlich eines Schritts zur Messung des pH-Werts, gefolgt von einem Schritt der Anpassung des pH-Werts der Zusammensetzung an einen gewünschten Wert, z.B. zwischen 6 und 8, oder etwa 7.

29. Verfahren zur Verpackung eines Impfstoffs, bei dem man: (a) einen Bulk-Impfstoff herstellt, der ein konjugiertes Saccharid umfasst; (b) den Gehalt an unkonjugiertem Saccharid in dem Bulk-Impfstoff durch ein Verfahren nach Anspruch 1 analysiert; (c) wahlweise den Bulk-Impfstoff hinsichtlich des pH-Werts und/oder anderer Eigenschaften analysiert; und, wenn die Ergebnisse von Schritt (b) und (c) darauf verweisen, dass der Bulk-Impfstoff für die klinische Verwendung akzeptabel ist, (d) den Impfstoff für die humane Verwendung ausgehend von dem Bulk-Impfstoff herstellt und verpackt.

## Revendications

1. Méthode d'analyse de la teneur en saccharides non conjugués d'un échantillon, comprenant les étapes (i) de passage de l'échantillon à travers un dispositif d'extraction sur phase solide pour séparer les saccharides conjugués des saccharides non conjugués et obtenir un spécimen comprenant des saccharides non conjugués séparés et (ii) d'analyse de la teneur en saccharides du spécimen pour donner la teneur en saccharides non conjugués de l'échantillon.

2. Méthode de préparation d'un échantillon pour l'analyse de la teneur en saccharides non conjugués, comprenant l'étape de passage de l'échantillon à travers un dispositif d'extraction sur phase solide pour séparer les saccharides conjugués des saccharides non conjugués.

3. Méthode selon la revendication 1 ou 2, comprenant l'étape de mesure de la teneur en saccharides totaux de l'échantillon.

4. Méthode selon l'une quelconque des revendications 1 à 3, dans laquelle le saccharide conjugué est un antigène saccharidique conjugué à une protéine de transport.

5. Méthode selon l'une quelconque des revendications 1 à 4, dans laquelle l'échantillon est un vaccin.

6. Méthode selon la revendication 5, dans laquelle le vaccin est un vaccin glycoconjugué.

7. Méthode selon la revendication 6, dans laquelle le vaccin glycoconjugué est un vaccin simple.

8. Méthode selon la revendication 6, dans laquelle le vaccin glycoconjugué est un vaccin combiné.

9. Méthode selon la revendication 8, dans laquelle le vaccin glycoconjugué combiné comprend un conjugué provenant du sérogroupe méningococcique C.

10. Méthode selon la revendication 9, dans laquelle le vaccin glycoconjugué comprend des mélanges de conjugués provenant de chacun des sérogroupes méningococciques C et Y.

11. Méthode selon la revendication 9, dans laquelle le vaccin glycoconjugué comprend des mélanges de conjugués provenant de chacun des sérogroupes méningococciques C, W135 et Y.

12. Méthode selon la revendication 9, dans laquelle le vaccin glycoconjugué comprend des mélanges de conjugués provenant de chacun des sérogroupes méningococciques A, C, W135 et Y.

13. Méthode selon la revendication 9, dans laquelle le vaccin glycoconjugué comprend des mélanges de conjugués provenant de chacun des sérogroupes méningococciques A et C.

14. Méthode selon l'une quelconque des revendications 9 à 13, comprenant l'étape d'analyse de la teneur en saccharides non conjugués du sérogroupe C de *N. meningitidis* de l'échantillon.

15. Méthode de séparation d'un composant saccharidique conjugué dans un échantillon d'un composant saccharidique non conjugué dans l'échantillon, comprenant l'étape de passage de l'échantillon à travers un dispositif d'extraction sur phase solide.

16. Utilisation d'un dispositif d'extraction sur phase solide pour séparer un composant saccharidique conjugué dans un échantillon d'un composant saccharidique non conjugué dans l'échantillon.

17. Méthode selon la revendication 15 ou utilisation selon la revendication 16, dans laquelle le saccharide conjugué est un antigène saccharidique conjugué à une protéine vecteur.

18. Méthode selon l'une quelconque des revendications 15 ou 17 ou utilisation selon la revendication 16 ou la revendication 17, dans laquelle l'échantillon est un vaccin.

19. Méthode ou utilisation selon la revendication 18, dans laquelle le vaccin est un vaccin glycoconjugué.

20. Méthode ou utilisation selon la revendication 19, dans laquelle le vaccin glycoconjugué est un vaccin simple.

21. Méthode ou utilisation selon la revendication 20, dans laquelle le vaccin glycoconjugué est un vaccin combiné.

22. Méthode ou utilisation selon la revendication 21, dans laquelle le vaccin glycoconjugué combiné comprend un conjugué provenant du sérogroupe méningococcique C.

23. Méthode ou utilisation selon la revendication 22, dans laquelle le vaccin glycoconjugué comprend des mélanges de conjugués provenant de chacun des sérogroupes méningococciques C et Y.

24. Méthode ou utilisation selon la revendication 22, dans laquelle le vaccin glycoconjugué comprend des mélanges de conjugués provenant de chacun des sérogroupes méningococciques C, W135 et Y.

25. Méthode ou utilisation selon la revendication 22, dans laquelle le vaccin glycoconjugué comprend des mélanges de conjugués provenant de chacun des sérogroupes méningococciques A, C, W135 et Y.

26. Méthode ou utilisation selon la revendication 22, dans laquelle le vaccin glycoconjugué comprend des mélanges de conjugués provenant de chacun des sérogroupes méningococciques A et C.

27. Méthode de libération d'un vaccin pour une utilisation par des médecins, comprenant les étapes consistant à : (a) fabriquer un vaccin comprenant un saccharide conjugué ; (b) analyser la teneur en saccharides non conjugués du vaccin par une méthode selon la revendication 1 ; et, si les résultats issus de l'étape (b) indiquent une teneur en saccharides acceptable pour une utilisation clinique, (c) libérer le vaccin pour une utilisation par des médecins.

28. Méthode de préparation d'une composition vaccinale, comprenant une étape d'analyse de la teneur en saccharides non conjugués du vaccin par une méthode selon la revendication 1, y compris une étape de mesure du pH, suivie d'une étape d'ajustement du pH de la composition à une valeur souhaitée, par exemple entre 6 et 8, ou environ 7.

29. Méthode de conditionnement d'un vaccin, comprenant les étapes consistant à : (a) fabriquer un vaccin à l'état brut contenant un saccharide conjugué ; (b) analyser la teneur en saccharides non conjugués dans le vaccin à l'état brut par une méthode selon la revendication 1 ; (c) éventuellement, analyser le vaccin à l'état brut pour le pH et/ou d'autres propriétés ; et, si les résultats issus de l'étape (b) et (c) indiquent que le vaccin à l'état brut est acceptable pour une utilisation clinique, (d) préparer et conditionner le vaccin pour une utilisation humaine à partir de l'état brut.
